# EUROPEAN PATENT APPLICATION

(11) **EP 4 443 360 A1**
(43) Date of publication of application: **09.10.2024**
(21) Application number: 24166958.9
(22) Date of filing: 27.03.2024
(51) Int. Cl.: G06Q 10/0639, A61B 5/16, G06Q 10/0635, A61B 5/0205

(54) **ORGANIZATIONAL CLASSIFICATION SYSTEM AND METHODS FOR THE PSYCHOSOCIAL MONITORING OF LEADERSHIP AND MANAGERIAL RISK**

(30) Priority: 03.04.2023 IT 202300006468
(71) Applicant: Di Lorenzo, Maria Rosaria, 00198 Roma (RM) (IT)
(72) Inventor: Di Lorenzo, Maria Rosaria, 00198 Roma (RM) (IT)
(74) Representative: Fiammenghi, Eva

(57) **Abstract**

Organizational classification systems and methods for the psychosocial monitoring of leadership and managerial risk (100) to monitor the socio-statistical-psychological state of the members (11) of a company/Public Administration or organization (10), and obtain an organizational profile, comprising:
- leadership style analysis tests (101) to classify members (11) through areas (102), factors (103) and personological features (104) defined in detail and identify their strengths and weaknesses;
- risk propensity tests (110) to classify the propensity, perception and assumption of business risk;
- control apparatus (120) to monitor the physical state of the members (11) during the tests;
- Web portal and/or application (130) through which to administer the tests, provide advice to optimize one's strengths and improve one's weaknesses through the aid of ad hoc training activities, monitor changes, verify satisfaction and suggest changes and upgrades in company dynamics.

## Description

### Field of the art

The invention relates to the field of statistical-sociological analysis applied to personological analyses of psychological leadership styles. Even more specifically, the invention is adapted to provide a system for statistical-sociological and psychological classification adapted to provide support in the social dynamics within the teams of any company, public administration or organization, both in horizontal and vertical terms. Leadership skills and the aforementioned social dynamics can also be increased by means of the identification (through a survey) and the development of skills in defining the sources of conflict, in its management and in the related negotiation.

### Prior art

Work psychology deals with the relationships between different working subjects and organizational contexts, highlighting the personal, interpersonal and situational factors which act in the construction of individual and collective relationships. Work psychology studies the behavior of people in a given work situation and in carrying out their professional activity. The goal of work psychology is to promote corporate and individual workplace well-being in a specific manner. Therefore, the occupational psychologist carries out various tasks within a company, all aimed at achieving corporate and individual well-being which will affect better work performance. Working as an occupational psychologist means applying psychological models and theories to the work environment to ensure good psychological conditions and promote the working identity of the person - also through career counseling interventions. The occupational psychologist specifically deals with personnel selection, training and development, career consultancy, skills development and specific skill learning, performance analysis and the relationship between employee and company. Furthermore, psychologists of organizations or companies analyze the functioning of work groups and relationships between groups and intervene to improve it, intervene on leadership to identify the effectiveness of managerial action; contribute to increasing the quality of trade union relations and negotiation processes; examine and intervene on the psychosocial factors that influence organizational functioning; cooperate so that organizational change processes have a sustainable impact on people's lives; contribute to the enrichment of internal and external communication systems, etc. The psychologist within a company or organization evaluates the current situation and the potential of the working subjects that constitute it. They thereby examine the effectiveness in implementing a series of strategies and intervene on any errors which could cause economic or relational damage over time.

There are many examples of private companies which deal with proposing solutions to problems of various kinds through business psychology and its various multiple forms of implementation.

An example is the subject of patent application CN107799165A of X. XU. It refers to a psychological assessment method based on virtual reality technology. The processing of the virtual scenario is performed on a psychological scale; a subject's responses, behaviors and physiological data are collected in real time; on the basis of the subject's response options, their psychological picture is completed; all the evaluated data are combined and inserted into a software program to reach a complete evaluation; with the psychological evaluation model, the result of the subject's psychological evaluation is compared with a medical card. According to the above-mentioned invention, based on the combination of VR, intelligent detection, big data analysis, artificial intelligence and other technologies with the traditional psychological evaluation method, the accuracy of the psychological evaluation is improved and medical resources are saved in an effective manner.

Another example is the subject of patent application US20140214709A1 of K.G. GREANY. It refers to apparatuses, methods and systems for the occupational performance assessment, (OPA). The aforementioned invention provides apparatuses, methods and systems for conducting an OPA which transforms the answers to the candidates' occupational tests and the inputs of the employers' professional objectives obtained through the administration of psychological tests, into professional evaluation results of the candidates. In an embodiment, the OPA provides a technology-based system approach provided by computers for the occupational psychological assessment for talent identification, acquisition, management, and support, which adapts to a user's responses to provide an individualized assessment. The OPA can also be integrated with mobile technology and multi-touch interfaces.

Another example is the subject of patent application US20180025303A1 of J.T. JANZ. It refers to a system and method for computerized predictive performance analyses based on natural language. The cited invention comprises the steps of: acquiring a video stream of an interviewee responding to a series of interview requests or a corpus of documents of a subject; analyzing the semantic content of the video stream or corpus; statistically processing, with at least an automated processor, the semantic content according to a correspondence of the interviewee's response or the subject's corpus with a set of classified exemplary responses, and classifying the interviewee or subject with respect to the context; and generating at least an output selectively dependent on the classification of the interviewee or subject.

The inventions reported so far by way of purely illustrative and non-exhaustive examples are representative of the inventive-technological classification available to date.

The object of the present invention is to propose a system and a method which comprises means for efficiently and reliably administering psychological and/or psycho/aptitude tests to members of a company or organization. The administration of the test aims to take a snapshot of the psychological state of the company members and their mood. The purpose of such a test is to ensure greater company efficiency, both in interpersonal relationships and in individual tasks, which may tend to be more suitable for individuals who fall under a particular psychological profile. The invention, in addition to solving the technical problem of corporate efficiency, guaranteeing a greater state of satisfaction and a pleasant working environment for individual workers, also solves the technical problem of guaranteeing the reliability of the psychological test administered, providing devices capable of monitoring the conditions of the individuals during the test, and indicating to professionals an estimate of the reliability of their answers.

Even more advantageously, the invention solves the technical problem of the continuous psychological monitoring of the workers of the company or organization, going beyond the instantaneous snapshot which a single psychological test can provide and thus guaranteeing a psychological monitoring system, capable of verifying the sentiment of the workers over time, and therefore offering a support service thereto and then also to the company or organization.

### Description of the invention

According to the present invention, an organizational psychological classification and monitoring system is created.

The system allows for the study and analysis of leadership styles and risk propensity within a company, a public or private organization or a group of subjects, the study of the diversity of the various members thereof, quantitative and qualitative analyses as well as a redesign of organizations and company dynamics with a view to optimizing results and improving performance. The invention proposes socio-statistical and psychological tests capable of providing an exact snapshot of the emotional state of the participating members of the company, public administration or organization at that precise moment. To overcome the technical problem linked to the time punctuality of this type of analysis, and so as to ensure a service which can be both analysis and monitoring at the same time, the invention entrusts the administration of these tests to a software system which re-runs them with a time frequency defined by the professional responsible for the monitoring. Lastly, so as to overcome the technical problem linked to the poor reliability which a psychological test can have on individuals in a particular emotional state, the invention adopts a control apparatus capable of establishing, through specific biometric sensors, the psycho-physical characteristics of the person undergoing the test.

The invention provides, within a web portal and/or an application, the possibility of following and/or booking courses. The courses are often recommended by the portal itself to allow the skills development of workers and leaders based on the results obtained in the psychological tests carried out.

The invention provides, in light of the results examined both from a statistical, psychological and managerial management point of view, reports where the company, Public Administration or organization involved collects information on the strengths and weaknesses of the team or company areas that participated. The reports on the analyses carried out provide indications on training courses adapted to fill the gaps which emerged by means of soft skills. In a separate report, the steps and peculiarities necessary to propose the soft skills necessary to strengthen the weaknesses found are outlined.

The courses made available are aimed at ensuring greater holistic well-being of the individual members and of the company, the Public Administration or the organization as a whole, and can be coaching courses, team coaching, counseling, training projects to allow to improve one's relationship with the management of their team, bioenergetic work, interventions aimed at both improving the strengths which have emerged and counteracting the weaknesses, strengthening relational and group aspects with particular attention to trust and the ability to build a path of responsibility and mutual respect for skills. Through theatrical exercises, improvisations, corporality, eliminating roles and starting over from looking, listening, energy, for emotional, psycho-attitudinal and behavioral rebalancing, courses relating to nutrition and diet, courses on phytotherapy, courses and workshops for the re-alignment of mind and body and many others. The courses section can also propose to company, Public Administration or organization workers the practice of activities which boost the motivation for change while keeping in mind the needs of followers and leaders. The tests are structured on the basis of in-depth research in the socio-psychological, organizational and management fields and allow companies or organizations and the parts/areas of expertise to receive socio-statistical profiling (monovariate, bivariate and multivariate), profiling on leadership styles analyzed according to areas, factors and personality characteristics, and profiling on analyses of managerial risk propensity within the company.

In the first instance, the system deals with identifying a representative sample of all the participating members of the company, Public Administration or organization; this operation is carried out by acquiring the personal data related to gender, age, educational qualifications, seniority of the professional role, type of contract, place of work, residence and others, according to the client's wishes, in a quantitative and qualitative analysis of the members of the company, Public Administration or organization. Subsequently, the system involves the administration of a test to analyze the personal leadership characteristics and subsequently a risk propensity test. The tests are compiled on a specific web portal and/or application, can be left pending and then continued, and guarantee data confidentiality through high security standards.

For the leadership styles analysis test, four fundamental areas are defined which are:
- structural area: related to the mastery of internal forces, the management of working groups and collegial bodies, where present, and the company vision;
- symbolic area: linked to the company vision, positioning with respect to the company vision and communication;
- political area: related to the valorization of resources, creativity, and the ability to negotiate and resolve conflicts;
- human resources area: the key characteristics of which are trust, attention towards employees and the valorization of resources.

In a preferred embodiment of the invention, each area in a tree diagram refers to ten factors and each factor to four personality characteristics, for a total of 40 items. A score is attributed to each item or characteristic so that the Web portal and/or the application, through a specific data collection and grouping algorithm, provides diamond graphs, for both areas, factors and characteristics of leadership styles, where the maximum and minimum points are indicated which represent the strengths and weaknesses of the group analyzed, so as to identify possible activities to improve poorly developed skills and/or to intervene more clearly on skills which have obtained a low score, where it is necessary within the company or organization that the interviewees remain in the interview position.

The graphs, accompanied by the human interpretation of experts in sociological statistics, in socio-organizational business analysis, constitute the reports delivered to the customer, according to the requests received and will be uploaded to the dedicated online portal in the specific customer report section.

The risk propensity test comprises, in the preferred embodiment of the invention, at least 12/18 items through which the degree of agreement with the propensity, perception and tendency to take risks is requested. Also in this case, the collection and grouping algorithm of the collected data and the report section, by interpolating the data from this test with those obtained from the leadership styles analysis test, are able to provide a forecast of the company's evolution and allow planning events aimed at optimizing processes and interactions within the company or organization.

The supporting hardware to be used is a heart rate monitor, pulse oximeter, thermal camera, and similar types.

Both tests are conducted envisaging the connection to the control apparatus. The control apparatus technology can also be included within the workstation of individual members of the company, Public Administration or organization. In fact, the apparatus comprises a video camera and a thermal detection unit which can be integrated into the webcam of the workstation computer, and then comprises a heartbeat sensor which can be integrated into the desk as a touch sensor on which to place the hand and/or a finger to detect blood pressure and heart rate.

This data is recorded during tests to evaluate possible altered psycho-physical states and to verify the reliability of the extracted results. The system requires that the members of the company or organization then monitor their biometric data with a certain time interval established by a professional responsible for the monitoring, so as to instruct the system itself about the standard personal biometric conditions and the biometric data of each individual. All the devices of the control apparatus send the recorded data wirelessly or in wired mode to the web portal and/or application to be analyzed with a specific analysis algorithm.

The tests are finally repeated with a predefined and customizable time frequency. Before carrying out a test, the system warns the user to connect to the control apparatus and, if the data detected is outside the standard biometric conditions, the system allows the tests to be postponed.

In a further embodiment of the invention, a survey can also be comprised which is adapted to identify and develop personal skills in defining sources of conflict, in their management and in the related negotiation.

The advantages offered by the present invention are evident in the light of the description presented thus far and will be even clearer thanks to the attached figures and the related detailed description.

### Description of the figures

The invention will be described hereinafter in at least a preferred embodiment by way of non-limiting example with the aid of the appended figures, in which:
- FIGURE 1 shows a general view of the organizational psychological classification and monitoring system 100;
- FIGURE 2 shows a view of the operating method 200 of said organizational psychological classification and monitoring system 100.

### Detailed description of the invention

The present invention will now be illustrated by way of a purely non-limiting or binding example, resorting to the figures which illustrate some embodiments with respect to the present inventive concept.

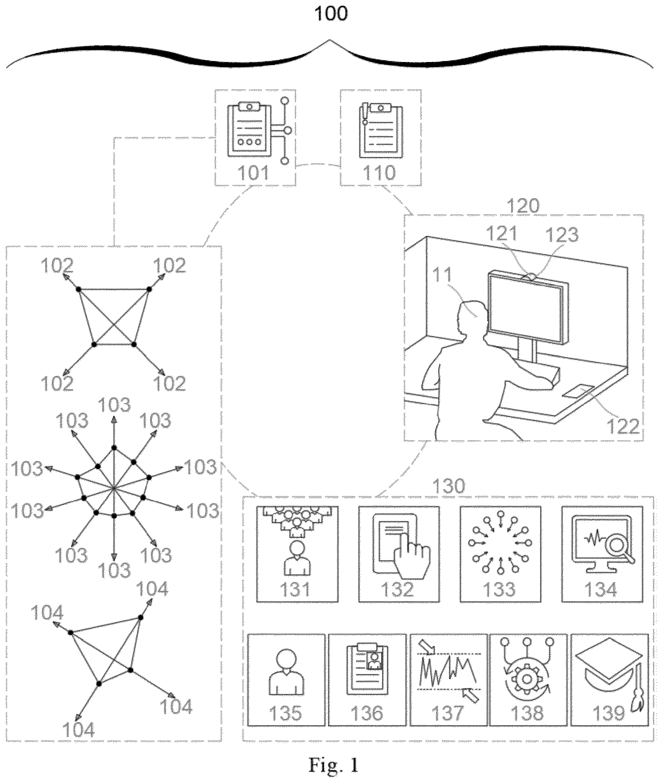

With reference to FIG. 1, a general view is shown of said organizational psychological classification and monitoring system 100 according to the present invention. In FIG. 1 as in the following description, the embodiment of the present invention currently considered the best is illustrated.

Said organizational psychological classification and monitoring system 100 is adapted to be used by at least a professional 1 for a company 10 or organization to photograph/monitor the emotional state of the members 11 regarding leadership styles and corporate risk management, and to obtain a precise and continuously evolving organizational profile with the personnel, and comprises at least a test of leadership 101, areas 102, factors 103, characteristics 104, at least a risk propensity test 110, at least a control apparatus 120, at least a video camera 121, at least a pulse sensor 122, at least a thermal detection unit 123, at least a web portal and/or application 130, at least a sample section 131, at least a test section 132, at least a data collection and grouping algorithm 133, at least a monitoring section 134, at least a user section 135, at least a report section 136, standard biometric conditions 137, at least a analysis algorithm 138, a courses section 139.

Said leadership style analysis test 101 is designed ad-hoc to classify said members 11 through areas 102, factors 103 and behavioral personological characteristics 104 specifically defined to ensure a detailed characterization and identify their strengths and weaknesses, while said risk propensity test 110 classifies the propensity, perception and assumption of business risk thereof.

Said control apparatus 120 is used to monitor the physical state of said members 11 during the administration of said leadership test 101 and at a regular interval defined by said professional 1, and to deduce the reliability and correspondence with reality thereof and comprises said video camera 121 adapted to film said members 11, said pulse sensor 122 which monitors the blood pressure and heart rate thereof and said thermal detection unit 123 which records the body temperature thereof. The data detected by said video camera 121, said pulse sensor 122 and said thermal detection unit 123 are processed by said analysis algorithm 138. Said courses section 139 is adapted to make courses available to said members 11 to follow directly online and/or the possibility of booking live courses, advising them based on the results of the tests carried out.

Said Web portal and/or application 130 is adapted to allow the administration of said leadership style analysis test 101 with a predefined and customizable frequency, adapted to provide indications useful to said members 11 to improve their strengths or improve their weaknesses through the aid of training activities targeted to the needs of the examined compartments and adapted to interact continuously with said members 11 to monitor their changes over time, verify their state of satisfaction, and suggest changes in the internal dynamics of the company, Public Administration or organization to optimize the conditions of well-being of the individual and the corporate, Public Administration or organizational community.

The Web portal and/or application 130 comprises said sample section 131 adapted to carry out a descriptive analysis of a sample of said members 11 representative of their entirety, said test section 132 adapted to allow the administration of said leadership test 101 and said risk propensity test 110 in connection with said control apparatus 120, said data collection and grouping algorithm 133 adapted to provide said professional 1 with a general picture indicative of the outcome of the tests, said monitoring section 134 is adapted to allow monitoring of the psychological conditions of said members 11, said user section 135 containing personal and company information, a history of the tests performed, and the parameters recorded with said control apparatus 120, and said report section 136 adapted to provide reports to said company 10 and to each of said corporate, Public Administration or organizational groupings or sections, or said members 11 related to the results of the tests and actions undertaken to improve the corporate, Public Administration or organizational set-up.

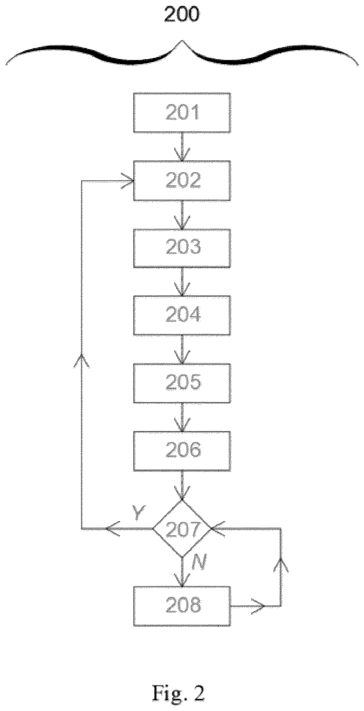

With reference to FIG. 2, it shows a view of said operating method 200 of said organizational classification and monitoring system 100. Said operating method 200 will be described with reference to FIGS. 1 and 2.

Said operating method 200 is adapted to exploit said organizational psychological classification and monitoring system 100 according to at least a sample definition step 201, a leadership style analysis step 202, an organizational risk analysis step 203, a data processing and display step 204, at least a monitoring step 205, a threshold definition step 206, at least a re-analysis step 207, a re-scheduling step 208.

Said sample definition step 201 envisages the definition of a sample so that it is representative of all said members 11 of said company, Public Administration or organization 10; the definition of the sample 201 takes place through said sample section 131 by carrying out a socio-personal, quantitative and qualitative analysis, and of the diversity of said members 11 by acquiring data relating to gender, age, educational qualification, seniority of the professional role, type of contract, place of work, residence and still others. The method continues with said leadership style analysis 202 conducted by administering said leadership test 101 to said members 11 selected in said sample definition step 201 through said test section 132. Said risk analysis step 203 is conducted by administering said risk propensity test 110 to said members 11 of the sample. Said data processing and display step 204 envisages, through said data collection and grouping algorithm 133 processing the data acquired in said leadership styles analysis step 202 and corporate, Public Administration or organizational risk analysis step 203 and then reporting them in reports through said report section 136.

In said monitoring step 205, each of said members 11 chosen in said sample definition step 201 is asked, by said Web portal and/or application 130, to measure their biometric parameters, through said control apparatus 120, subsequently, in said threshold definition step 206, threshold values relating to the standard biometric conditions 137 are defined for the biometric parameters measured by said control apparatus 120. The method 200 then continues with said re-analysis step 207 which is adapted to provide a positive *Y* or negative *N* result based on the parameters measured by said control apparatus 120. When the biometric parameters fall within standard biometric conditions 137 the outcome is positive Y and the method 200 starts again from said leadership style analysis step 202. When the biometric parameters measured by said control apparatus 120 are below or above said standard biometric conditions 137, the outcome is negative N and the method 200 proceeds with a re-scheduling step 208 of the tests in which another date is established for the tests to be carried out.

Finally, it is clear that modifications, additions or variations that are obvious to a person skilled in the art can be made to the invention described so far, without thereby departing from the scope of protection provided by the attached claims.

## Claims

1. Organizational psychological classification and monitoring system (100), adapted to be used by at least a professional (1) for a company, public administration or organization (10) to monitor the emotional state of members (11) in the analysis of leadership and propensity, assumption and perception of company risk, and obtain a precise and constantly evolving organizational profile with personnel **characterized in that** it comprises:
- at least a leadership style analysis test (101) denoting socio-statistical, psychological and corporate risk features designed ad-hoc to classify said members (11) through areas (102), factors (103) and behavioral personological features (104) specifically defined to ensure a detailed characterization and identify strengths and weaknesses;
- at least a risk propensity test (110) adapted to frame the propensity, perception and assumption of business risk by said members (11);
- at least a control apparatus (120) adapted to monitor the physical state of said members (11) during the administration of said leadership style analysis test (101) and said risk propensity test (110) and on a defined regular basis by said professional (1), and to deduce his/her reliability and correspondence with reality;
- at least a web portal and/or application (130) adapted to allow the administration of said leadership style analysis test (101) and of said risk propensity test (110) with a predefined and customizable frequency, adapted to provide indications useful to said members (11) to improve their strengths and convert weaknesses through targeted activities emerging from the survey performed and adapted to interact continuously with said members (11) to monitor changes over time, check their status of satisfaction, and suggest changes in the internal dynamics of the organization;
said control apparatus (120) comprising:
- at least a video camera (121) adapted to film said members (11);
- at least a pulse sensor (122) adapted to monitor the pressure and heart rate of said members (11);
- at least a thermal detection unit (123) adapted to monitor the body temperature of said members (11);
said web portal and/or application (130) comprising:
- at least a sample section (131) adapted to carry out a descriptive analysis of a sample of said members (11) representative of their entirety;
- at least a test section (132) adapted to allow the administration of said leadership style analysis test (101) and said risk propensity test (110) in connection with said control apparatus (120);
- at least a data collection and grouping algorithm (133) adapted to provide said professional (1) with a general picture indicative of the outcome of said leadership style analysis test (101) and said risk propensity test (110);
- at least a monitoring section (134) adapted to allow monitoring of the psychological conditions of said members (11);
- at least a user section (135) relating to each of said members (11) adapted to contain the personal and company data/of the public administration or organization, a history of the tests performed, and the parameters recorded with said control apparatus (120);
- at least a report section (136) adapted to provide reports to said company, public administration or organization (10) and to each participating area/sector of said members (11) relating to the results of said leadership test (101) and said risk propensity test (110) and actions taken to improve the corporate, public administration or organization set-up;
- a courses section (139) adapted to make courses available to said members (11) to be followed directly online and/or the possibility of booking live courses; said courses section (139) adapted to present particular courses to said members (11) also on the basis of the results of said leadership test (101) and said risk propensity test (110).

2. Organizational psychological classification and monitoring system (100), according to the preceding claim 1, **characterized in that** said data collection and grouping algorithm (133) is adapted to characterize the data, obtained with said leadership style analysis test (101) carried out on said members (11), through at least four areas (102), ten factors (103) and four personological features (104); each of said areas (102), of said factors (103) and of said personological features (104) is indicative of particular social and work skills which are characterized through a score; said data collection and grouping algorithm (133) adapted to provide companies, public administrations or organizations to which professionals (1) belong, as employees or non-employees, indications relating to the strengths and weaknesses of said members (11) and to ensure the possibility of identifying possible activities to improve underdeveloped skills and/or to intervene more clearly on skills that have obtained a low score.

3. Organizational psychological classification and monitoring system (100), according to the preceding claim 1 or 2, **characterized in that** said risk propensity test (110) comprises at least twelve items through which said members (11) indicate their degree of agreement in relation to the propensity, perception and tendency to take risk; said data collection and grouping algorithm (133) adapted to relate the degree of agreement expressed by said members (11) in relation to each item of said risk propensity test (110) to particular personal feature which, connected to said areas (102), said factors (103) and said features (104) detected with said leadership test (101) are able to provide a forecast of the evolution of the company, the public administration or the organization and allow the planning of events aimed at the optimization of processes and internal interactions of said company/public administration or organization (10).

4. Organizational psychological classification and monitoring system (100), according to any one of the preceding claims, **characterized in that** said control apparatus (120) is integrated in the technology present on the workstation of each of said members (11) and/or is a completely or partially independent hardware and software apparatus; said video camera (121) and said thermal detection unit (123) adapted to be integrated into the webcam of the computer present in the workstation of each of said members (11); said pulse sensor (122) adapted to be integrated in the desk of the workstation of each of said members (11) as a touch sensor capable of detecting pressure and heartbeat through contact with the hand and/or a finger.

5. Organizational psychological classification and monitoring system (100), according to any one of the preceding claims, **characterized in that** said video camera (121), said pulse sensor (122) and said thermal detection unit (123) are adapted to send data recorded to said web portal and/or application (130) in wireless or wired mode to have them analyzed with a suitable analysis algorithm (138).

6. Organizational psychological classification and monitoring system (100), according to any one of the preceding claims, **characterized in that** said user section (135) is adapted to request from said members (11), after definition by said professional (1) of the time interval between one request and another, to provide said control apparatus (120) with their own biometric data, to store them and determine the standard biometric conditions (137) for each of said members (11).

7. Organizational psychological classification and monitoring system (100), according to any one of the preceding claims, **characterized in that** said monitoring section (134) is adapted to set dates on which said members (11) are subjected to said leadership style analysis test (101) and to send appropriate notifications; said monitoring section (134) adapted to communicate with said user section (135) in order to analyze the emotional-psycho-physical state of each of said members (11) at the moment in which he/she undergoes said leadership style analysis test (101) and said risk propensity test (110); said monitoring section (134) adapted to allow the postponement of the administration of said leadership test (101) and said risk propensity test (110) when said members (11) are in an emotional-psycho-physical state different from that associated with these standard biometric conditions (137).

8. Organizational psychological classification and monitoring system (100), according to any one of the preceding claims, **characterized in that** it provides sensitive data protection systems with high security standards to guarantee the privacy of said members (11).

9. Method of operation (200) of said organizational psychological classification and monitoring system (100), **characterized in that** it uses the system according to any one of the preceding claims **and in that** it comprises the following steps:
- defining the sample (201) so that it is representative of all said members (11) of said company/public administration or organization (10); the definition of the sample (201) takes place through said sample section (131) by carrying out a socio-personal, quantitative and qualitative analysis, and of the diversity of said members (11) by acquiring data relating to gender, age, educational qualification, seniority of the professional role, type of contract, place of work, residence and others;
- leadership style analysis (202) conducted by administering said leadership test (101) to said members (11) selected in said sample definition step (201) through said test section (132);
- risk analysis (203) conducted by administering said risk propensity test (110) to said members (11) selected in said sample definition step (201) through said test section (132);
- data processing and display (204) step in which the data acquired in said leadership style analysis (202) and risk analysis (203) steps are processed through said data collection and grouping algorithm (133) and then reported in reports through said report section (136);
- monitoring (205), step in which each of said members (11) selected in said sample definition step (201) is requested, by said Web portal and/or application (130), to measure their own biometric parameters, through said control apparatus (120);
- definition of thresholds (206) step in which, following said monitoring step (205) which is repeated over time, threshold values relating to the standard biometric conditions (137) are defined for the biometric parameters measured by said control apparatus (120);
- re-analysis (207), this step is adapted to provide a positive (*Y*) or negative (N) result based on the parameters measured by said control apparatus (120); when the biometric parameters fall within the standard biometric conditions (137) the outcome is positive (*Y*) and the method (200) restarts from said leadership style analysis step (202); when the biometric parameters measured by said control apparatus (120) are below or above said standard biometric conditions (137), the outcome is negative (*N*) and the method (200) proceeds with a step of re-scheduling (208) the tests in which another date is established for the execution of the tests.

10. Method (200) according to the preceding claim 9, **characterized in that** said steps of leadership style analysis (202) and risk analysis (203) are carried out after connecting said members (11) to the devices of said control apparatus (120).

11. Method (200) according to the preceding claim 9 or 10, **characterized in that** it comprises a step of administering a survey adapted to identify and possibly develop and increase the ability to define the sources of conflict, in its management and in the relative negotiation.
